(19) Europäisches Patentamt
European Patent Office
Office européen des brevets

(11) **EP 4 783 791 A1**

(12) **EUROPEAN PATENT APPLICATION**
published in accordance with Art. 153(4) EPC

(43) Date of publication:
29.07.2026 Bulletin 2026/31

(21) Application number: 24868059.7

(22) Date of filing: 28.08.2024

(51) International Patent Classification (IPC):
*H10H 20/855* (2025.01)    *A61N 5/06* (2006.01)
*C09K 11/62* (2006.01)    *G02B 5/02* (2006.01)
*G02B 5/20* (2006.01)    *H10H 20/00* (2025.01)
*H10H 20/851* (2025.01)

(52) Cooperative Patent Classification (CPC):
A61N 5/06; C09K 11/62; G02B 5/02; G02B 5/20

(86) International application number:
PCT/JP2024/030777

(87) International publication number:
WO 2025/062979 (27.03.2025 Gazette 2025/13)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 21.09.2023 JP 2023156367

(71) Applicant: Panasonic Intellectual Property
Management Co., Ltd.
Kadoma-shi, Osaka 571-0057 (JP)

(72) Inventors:
• IWATA, Koki
kadoma-shi, Osaka 571-0057 (JP)
• NITTA, Mitsuru
kadoma-shi, Osaka 571-0057 (JP)
• OSHIO, Shozo
kadoma-shi, Osaka 571-0057 (JP)
• MORI, Yusuke
kadoma-shi, Osaka 571-0057 (JP)

(74) Representative: Müller-Boré & Partner
Patentanwälte PartG mbB
Friedenheimer Brücke 21
80639 München (DE)

(54) **NEAR-INFRARED LIGHT-EMITTING DEVICE AND ELECTRONIC APPARATUS**

(57) A near-infrared light-emitting device (1) includes at least one solid-state light-emitting element (10) emitting primary light (11) having a maximum spectral intensity within a wavelength range of 380 nm or more, and less than 700 nm; a light diffuser (20) that diffuses the primary light (11) to emit diffused light (21); and a wavelength converter (30) including a near-infrared phosphor (31) that absorbs the diffused light (21) and converts the diffused light (21) into near-infrared rays having a maximum fluorescence intensity within a wavelength range of 700 nm or more, and less than 2500 nm, and is configured to emit output light (50) that contains the near-infrared rays as a light component.

FIG. 1

# Description

## TECHNICAL FIELD

The present invention relates to a near-infrared light-emitting device and electronic equipment.

## BACKGROUND ART

Conventionally, a light emitting device is known, which emits uniform white light by diffusing light emitted from a solid-state light-emitting element with a light diffuser. Patent Literature 1 discloses a lighting device including a substrate, a plurality of white LED elements mounted on the substrate, a plurality of near infrared LED elements mounted on the substrate, and a diffusion transmission case arranged to face the plurality of white LED elements and the plurality of near-infrared LED elements. Blue LED elements emit blue light, and a diffusing agent of yellow phosphor is dispersed in the transmission diffusion case. Therefore, the blue light emitted from the blue LED elements is diffused through the transmission diffusion case to emit white light.

## CITATION LIST

## PATENT LITERATURE

Patent Literature 1: Japanese Patent Application Publication No. 2015-076872

## SUMMARY OF INVENTION

However, the light emitted from the solid-state light-emitting element has a strong directivity, and near-infrared rays have a property of easily penetrating a substance in comparison with visible light rays. It is considered that the near-infrared rays emitted from a light output surface penetrate more easily than the visible light rays in the transmission diffusion case in Patent Literature 1, and that the diffusion effect is weakened. Therefore, in the lighting device disclosed in Patent Literature 1, the intensity distribution of the near-infrared rays within the light output surface becomes inhomogeneous, and there is a possibility that the near-infrared rays cannot be homogenously irradiated to the object to be irradiated.

The present invention has been made in view of such problems of the conventional technology. An object of the present invention is to provide a near-infrared light-emitting device that emits near-infrared rays having a homogeneous intensity distribution in a light output surface, and electronic equipment using the near-infrared light-emitting device.

According to an embodiment of the present invention, the problems described above are solved by a near-infrared light-emitting device including at least one solid-state light-emitting element emitting primary light having a maximum value of spectral intensity within a wavelength range of 380 nm or more, and less than 700 nm. The near-infrared light-emitting device includes a light diffuser that diffuses the primary light to emit diffused light, and a wavelength converter including a near-infrared phosphor that absorbs the diffused light and converts the diffused light into near-infrared rays having a maximum fluorescence intensity within a wavelength range of 700 nm or more, and less than 2500 nm. The near-infrared light-emitting device is configured to emit output light containing the near-infrared rays as a light component.

Electronic equipment according to an embodiment of the present invention includes a near-infrared light-emitting device.

## BRIEF DESCRIPTION OF DRAWINGS

[FIG. 1] FIG. 1 is a schematic diagram illustrating an example of a near-infrared light-emitting device according to an embodiment.
[FIG. 2] FIG. 2 is a schematic diagram illustrating an example of a structure of a spectroscopic device according to an embodiment.
[FIG. 3] FIG. 3 is a schematic diagram illustrating an example of a structure of a spectroscopic device according to another embodiment.
[FIG. 4 ] FIG. 4 is a schematic diagram illustrating an evaluation device for imaging output light of a near-infrared light-emitting device according to an example.
[FIG. 5] FIG. 5 is a fluorescence spectrum of a phosphor according to an embodiment.
[FIG. 6] FIG. 6 is a schematic diagram illustrating an evaluation device for imaging output light of a near-infrared light-emitting device according to Comparative Example 1.
[FIG. 7] FIG. 7 is a schematic diagram illustrating an evaluation device for imaging output light of a near-infrared light-emitting device according to Comparative Example 2.
[FIG. 8] FIG. 8 is a photograph of an output light image of a near-infrared light-emitting device according to an embodiment.
[FIG. 9] FIG. 9 is a photograph of an output light image of a near-infrared light-emitting device according to Comparative Example 1.
[FIG. 10] FIG. 10 is a photograph of an output light image of a near-infrared light-emitting device according to Comparative Example 2.
[FIG. 11] FIG. 11 is a graph illustrating the relationship between blue LED power and fluorescent power.
[FIG. 12] FIG. 12 is a graph illustrating the relationship between blue LED power and SWIR (shortwave infrared) conversion efficiency.

DESCRIPTION OF EMBODIMENTS

Hereinafter, a near-infrared light-emitting device, electronic equipment, a sensing system, and a near-infrared spectroscopy according to the present embodiment will be described in detail with reference to the drawings. Note that the dimensional ratios in the drawings are exaggerated for the sake of explanation and may differ from the actual ratios.

Near-infrared light-emitting device

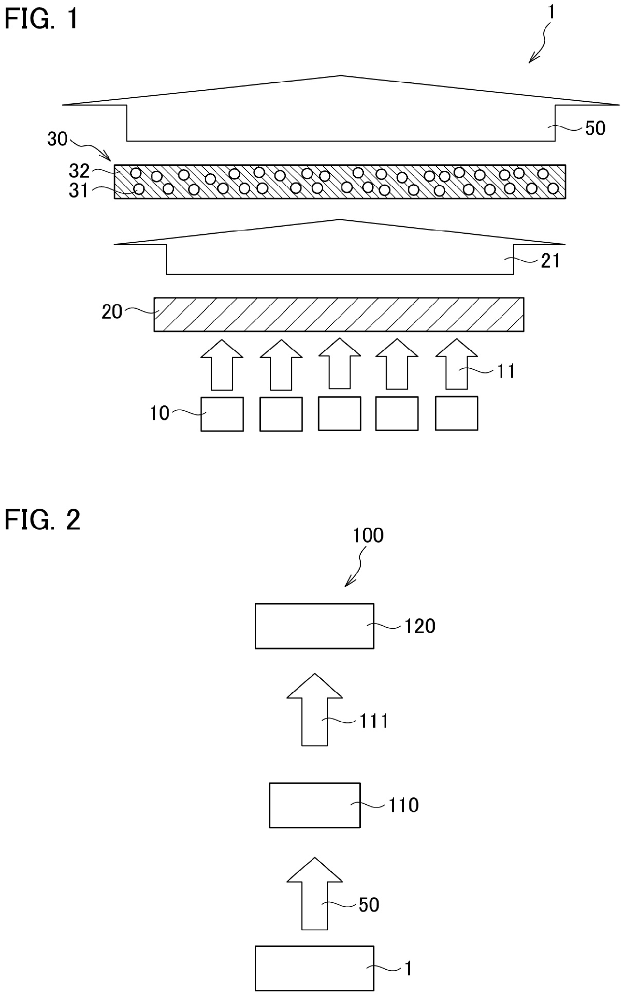

As illustrated in FIG. 1, a near-infrared light-emitting device 1 according to the present embodiment includes at least one solid-state light-emitting element 10, a light diffuser 20, and a wavelength converter 30. The at least one solid-state light-emitting element 10 emits primary light 11 having a maximum value of spectral intensity within a wavelength range of 380 nm or more, and less than 700 nm. The light diffuser 20 diffuses the primary light 11 to emit diffused light 21. The wavelength converter 30 includes a near-infrared phosphor 31 that absorbs the diffused light 21, and converts the diffused light 21 into near-infrared rays having a maximum fluorescence intensity within a wavelength range of 700 nm or more, and less than 2500 nm. The near-infrared light-emitting device 1 is configured to emit output light 50 containing near-infrared rays as a light component. Note that the output light 50 may include the primary light 11.

With this configuration, the primary light 11, which includes visible light with a strong directivity, is emitted from the solid-state light-emitting element 10, and can be diffused by using an orthodox light diffuser 20 having a function of diffusing visible light. Further, the diffused light 21 obtained by diffusing the primary light 11 is absorbed by the near-infrared phosphor 31, and the diffused light 21 is converted into near-infrared rays. Therefore, near-infrared ray having a homogeneous intensity distribution in a light output surface due to a light scattering effect can be emitted from a relatively large area. This allows the near-infrared light-emitting device 1 according to this embodiment to emit near-infrared rays having a homogeneous intensity distribution in a light output surface. The near-infrared light-emitting device 1 according to the present embodiment is excellent in industrial productivity because an orthodox solid-state light-emitting element 10 and an orthodox light diffuser 20 can be used.

Further, the near-infrared light-emitting device 1 according to the present embodiment can emit the output light 50 including near-infrared rays as a light component by using the solid-state light-emitting element 10 using visible light, without using an expensive solid-state light-emitting element using near-infrared rays. Therefore, the manufacturing cost of the near-infrared light-emitting device 1 can be reduced. In addition, the near-infrared light-emitting device 1 according to the present embodiment diffuses light by using the light diffuser 20, the number of solid-state light-emitting elements 10 to be used can be reduced. Hereinafter, each component will be described in detail.

In this specification, light having a wavelength of 380 nm or more, and less than 700 nm, is defined as visible light, and light having a wavelength of 700 nm or more, and less than 2500 nm, is defined as near-infrared light. In this specification, visible light is also referred to as visible light rays, and near-infrared light is also referred to as near-infrared rays.

Solid-state light-emitting element 10

The solid-state light-emitting element 10 is configured to emit the primary light 11. The solid-state light-emitting element 10 is an element for converting electricity into light. When the solid-state light-emitting element 10 is driven by supplying electric power, it converts supplied electric energy into light energy. Then, the converted light energy is emitted from a light extraction surface to be the primary light 11 (excitation light). The solid-state light-emitting element 10 may include, for example, a light emitting diode (LED) or a laser diode.

The at least one solid-state light-emitting element 10 may be configured as one solid-state light-emitting element 10, or a plurality of solid-state light-emitting elements 10 as illustrated in FIG. 1. The plurality of solid-state light-emitting elements 10 may emit the primary light 11 in the same direction. The near-infrared light-emitting device 1 is advantageous as a surface-emitting type near-infrared light-emitting device 1 because the area of the light output surface is large. The number of the solid-state light-emitting elements 10 may be 2 or more, 5 or more, 9 or more, 25 or more, 36 or more, 49 or more, 64 or more, 81 or more, or 100 or more. The number of the solid-state light-emitting elements 10 may be 100 or less, 81 or less, 64 or less, 49 or less, 36 or less, 25 or less, 16 or less, or 9 or less.

The plurality of solid-state light-emitting elements 10 may be arranged at equal intervals. The near-infrared light-emitting device 1 configured in this manner can emit the primary light 11 at equal intervals. Therefore, visible light diffused by the light diffuser 20 can also be emitted at equal intervals. As a result, the surface-emitting type near-infrared light-emitting device 1, which emits homogeneous near-infrared rays over a wide area, can be provided.

The plurality of solid-state light-emitting elements 10 may be arranged at an average interval of less than 100 cm. When the plurality of solid-state light-emitting elements 10 are arranged close to each other in this manner, intensity unevenness in any plane of the primary light 11 can be further reduced. Therefore, the diffused light 21 (visible light) emitted from the light diffuser 20 can be homogenized to emit homogeneous near-infrared rays. The distance between the plurality of solid-state light-emitting elements 10 may be less than 30 cm, less than 3 cm, or less than 1 cm on average. The distance between the plurality of solid-state light-emitting elements 10 may be 1 cm or more on average. The solid-state light-emitting elements 10 may be arranged in one dimension to form a one-dimensional array, and may be used as a line-emitting type near-infrared light-emitting device 1. The solid-state light-emitting elements 10 may be arranged in two dimensions to form a two-dimensional array, and may be used as the surface-emitting type near-infrared light-emitting device 1. The distance of less than 100 cm on average means that the average distance between most adjacent solid-state light-emitting elements 10 is less than 100 cm.

The primary light 11 has a maximum spectral intensity within a wavelength range of 380 nm or more, and less than 700 nm. In this manner, the primary light 11 in a wavelength region from violet to red can be utilized by the orthodox solid-state light-emitting elements 10 including a light emitting layer exhibiting light emission transition in a type of direct transition, which can be expected to increase efficiency or output. Therefore, it is possible to provide the near-infrared light-emitting device 1 with high industrial productivity without requiring advanced technology.

The primary light 11 may be visible light having a maximum spectral intensity within a wavelength range of 440 nm or more, and less than 660 nm. Specifically, the primary light 11 may be visible light having a maximum spectral intensity within a wavelength range of either blue having a wavelength range of 440 nm or more, and less than 480 nm, or red having a wavelength range of 620 nm or more, and less than 660 nm. When the primary light 11 has a maximum spectral intensity within a wavelength range of 440 nm or more, and less than 480 nm, a blue LED that is easy to procure with high output power and high efficiency can be used. When the primary light 11 has a maximum spectral intensity within a wavelength range of 620 nm or more, and less than 660 nm, a red LED that is easy to procure with high output power and high efficiency can be used.

Light diffuser 20

The light diffuser 20 diffuses the primary light 11 to emit the diffused light 21. Near-infrared rays have a property of penetrating substances more easily than visible light. Therefore, the light diffusion for near-infrared rays requires an advanced technology. When such a technology is introduced, the manufacturing cost tends to increase, and the industrial use of the technology may be hindered. However, in the near-infrared light-emitting device 1 according to this embodiment, the light diffuser 20 diffuses primary light 11, including visible light. Therefore, it is possible to provide the near-infrared light-emitting device 1 that emits near-infrared rays having a homogeneous intensity distribution in a light output surface, without introducing the advanced technology described above.

The light diffuser 20 may transmit primary light 11 to emit diffused light 21. For example, the light diffuser 20 may mainly contain a resin, especially a translucent resin. The light diffuser 20 configured in this manner is easy to mold and process, and there are many technical choices for materials and methods. For this reason, the near-infrared light-emitting device 1 has high industrial productivity, and can easily respond to customer demands. It should be noted that the light diffuser 20 mainly containing a resin means that the light diffuser 20 contains a resin at 50% by volume or more. The light diffuser 20 may contain a resin at 60% by volume or more, 70% by volume or more, 80% by volume or more, 90% by volume or more, or 99% by volume or more. The light diffuser 20 may contain only resin. The light diffuser 20 may be a diffusion plate or a light guide plate.

Note that the light guide plate is a member that is processed to emit light from a light emitting surface. The light guide plate may be provided, for example, with at least one of a concave portion or a convex portion reflecting the primary light 11 on a processing surface which is a surface opposite to the light emitting surface. The primary light 11 may be guided from an end portion of the light guide plate in a direction perpendicular to the light emitting surface, reflected on the concave portion or the convex portion of the processing surface, and output from the light emitting surface. With this embodiment, the output light 50 containing near-infrared rays as a light component can be also irradiated over a wide range. Therefore, the amount of the solid-state light-emitting elements 10 to be used can be reduced. In addition, a near-infrared LED, which is generally more expensive than a blue LED, does not need to be used, so that an inexpensive near-infrared light-emitting device 1 can be manufactured using only a small amount of the solid-state light-emitting elements 10 and a general light guide plate.

The light diffuser 20 may include, for example, a matrix and a plurality of diffused particles dispersed in the matrix. The matrix may mainly contain a resin, particularly a translucent resin. The content of the resin contained in the matrix may be 50% by volume or more, 60% by volume or more, 70% by volume or more, 80% by volume or more, 90% by volume or more, or 99% by volume or more. The content of the resin contained in the matrix may be 90% by volume or less, 80% by volume or less, 70% by volume or less, or 60% by volume or less. The diffused particles can diffuse light passing through the light diffuser 20 by scattered reflection. The diffused particles may contain at least one of inorganic particles or organic particles.

The inorganic particles may contain at least one metal element selected from the group consisting of alkali metals, alkaline earth metals, transition metals, base metals, and semimetals. In this specification, the alkali metals include lithium, sodium, potassium, rubidium, cesium, and francium. The alkaline earth metals include beryllium and magnesium in addition to calcium, strontium, barium, and radium. The base metals include aluminum, zinc, gallium, cadmium, indium, tin, mercury, thallium, lead, bismuth, and polonium. The semimetal includes boron, silicon, germanium, arsenic, antimony, and tellurium. The inorganic particles may contain at least one selected from the group consisting of metals, metal salts, oxides, hydroxides, nitrides, borides, carbonates, fluorides, carbides, and sulfides. The diffused particles may contain, for example, silica.

The organic particles may contain resin particles. The resin contained in the resin particles may contain at least one selected from the group consisting of polyethylene, polypropylene, polyester, acrylic resin, polycarbonate, polystyrene, epoxy resin, and polyurethane.

The average particle diameter of the diffused particles is not particularly limited, and may be 0.5 μm or more, 1 μm or more, 3 μm or more, or 5 μm or more. The average particle diameter of the diffused particles may be 50 μm or less, 30 μm or less, 20 μm or less, or 10 μm or less. The average particle diameter of the diffused particles is calculated as an average value of particle diameters of particles observed in several to several tens of fields of view using an observation means such as a scanning electron microscope (SEM) or a transmission electron microscope (TEM).

The content of the matrix contained in the light diffuser 20 may be 10% by volume or more, 20% by volume or more, 30% by volume or more, 40% by volume or more, 50% by volume or more, 60% by volume or more, 70% by volume or more, 80% by volume or more, or 90% by volume or more. The content of the matrix contained in the light diffuser 20 may be 90% by volume or less, 80% by volume or less, 70% by volume or less, 60% by volume or less, 50% by volume or less, 40% by volume or less, 30% by volume or less, or 20% by volume or less.

The content of diffused particles contained in the light diffuser 20 may be 10% by volume or more, 20% by volume or more, 30% by volume or more, 40% by volume or more, 50% by volume or more, 60% by volume or more, 70% by volume or more, 80% by volume or more, or 90% by volume or more. The content of diffusion particles contained in the light diffuser 20 may be 90% by volume or less, 80% by volume or less, 70% by volume or less, 60% by volume or less, 50% by volume or less, 40% by volume or less, 30% by volume or less, or 20% by volume or less.

The light diffuser 20 may have irregularities on its surface, and diffuse light based on the irregularities. The pitch and height of the irregularities, the density of the irregularities, or the like are not particularly limited as long as light can be diffused, and can be suitably adjusted. The light diffuser 20 may have irregularities only on one of a light incident surface or a light emitting surface, or may have irregularities on both surfaces. The method of forming the irregularities is not particularly limited, and the surface of the substrate may be roughened or a member having irregularities may be provided on the surface of the substrate.

The light diffuser 20 need not include a phosphor. For example, the light diffuser 20 need not include a phosphor emitting fluorescence having a fluorescence peak within a wavelength range of 700 nm or more, and less than 2500 nm. For example, the diffused light 21 may include light having a maximum value of spectral intensity within a wavelength range of 380 nm or more, and less than 700 nm, and need not include near-infrared rays converted by the near-infrared phosphor 31. In the near-infrared light-emitting device 1 configured in this manner, the light diffuser 20 does not emit near-infrared rays originating from a phosphor. Therefore, the spectral distribution of the output light 50 can be simplified, and fluorescence interference can be mitigated between different types of phosphors. Further, the structure of the light diffuser 20 can be simplified, so that the near-infrared light-emitting device 1 with high industrial productivity can be provided without requiring advanced technology or a large number of man-hours. The light diffuser 20 need not include a red phosphor emitting fluorescence having a fluorescence peak within a wavelength range of 600 nm or more, and 660 nm or less. The diffused light 21 may have the same light component as the primary light 11.

The light diffuser 20 is not particularly limited in shape, and may be formed in a shape of a plate or a sheet. In this manner, the light diffuser 20 has a shape that extends horizontally with respect to a thickness direction. Therefore, the wavelength converter 30 can be irradiated with the diffused light 21 emitted from the light diffuser 20 over a wide area.

The light diffuser 20 may include a flat light emitting surface to emit the diffused light 21. In this manner, the surface of the wavelength converter 30 facing the light diffuser 20 becomes flat. Therefore, when the light diffuser 20 and the wavelength converter 30 are bonded to be integrated, it is possible to obtain a thin near-infrared light-emitting device 1. The flat light emitting surface means that the surface does not have irregularities caused by curvature.

Wavelength converter 30

The wavelength converter 30 includes the near-infrared phosphor 31. The near-infrared phosphor 31 absorbs the diffused light 21, and emits near-infrared rays having a maximum fluorescence intensity within a wavelength range of 700 nm or more, and less than 2500 nm.

The near-infrared phosphor 31 may emit near-infrared rays having a maximum fluorescence intensity within a wavelength range of 700 nm or more, and less than 1000 nm. Fluorescence having a light component in this wavelength range is suitable for detecting characteristic absorption bands of stretching vibrations of N-H, C-H, and O-H. Fluorescence emitted from the near-infrared phosphor 31 may have a maximum fluorescence intensity within a wavelength range of 700 nm or more, and 900 nm or less; or 780 nm or more, and 860 nm or less.

The near-infrared phosphor 31 may emit near-infrared rays having a maximum fluorescence intensity within a wavelength range of 1000 nm or more, and less than 2500 nm. Fluorescence having a light component in this wavelength range is suitable for evaluation of food quality for fresh fish, meat, or the like, and is also suitable for analysis of resin components for sorting out plastics. The fluorescence emitted from the near-infrared phosphor 31 may have a maximum fluorescence intensity of 1050 nm or more, 1100 nm or more, 1150 nm or more, or 1200 nm or more. The fluorescence emitted from the near-infrared phosphor 31 may have a maximum fluorescence intensity of 1600 nm or less, 1500 nm or less, 1400 nm or less, 1300 nm or less, or 1250 nm or less.

Luminance saturation has been examined for a near-infrared phosphor that emits near-infrared rays having a maximum fluorescence intensity within a wavelength range of 1000 nm or more, and less than 2500 nm, and it was found that luminance saturation tends to occur in the near-infrared phosphor. For example, in the near-infrared phosphor, a luminance saturation constant representing the influence of luminance saturation was 0.1 or more, and less than 0.8. However, the wavelength converter 30 according to the present embodiment uses light of a relatively low output via the light diffuser 20 as excitation light. Therefore, the influence of luminance saturation can be reduced.

The luminance saturation constant is expressed by the following formula.

$$I = \alpha I_{uv}^{\gamma}$$

In the above formula, I represents luminance of emitted light, $I_{uv}$ represents excitation intensity, $\alpha$ represents a constant, and $\gamma$ represents luminance saturation constant. Further, the luminance saturation constant $\gamma$ is a positive number of one or less, and when the luminance saturation constant $\gamma$ is one, it means that there is no luminance saturation.

A ratio of minimum intensity to maximum intensity is preferably 20% or more, and 100% or less in an excitation spectrum having a wavelength of 400 nm or more, and less than 700 nm in the near-infrared phosphor 31. The near-infrared phosphor 31 configured in this manner is not easily affected by the wavelength and spectrum of the primary light 11, and can be easily adapted to all types of LEDs such as blue LEDs, red LEDs, and white LEDs. Therefore, the near-infrared phosphor has high versatility.

As the near-infrared phosphor 31, inorganic phosphors known for near-infrared light sources can be used. Specifically, the near-infrared phosphor 31 may contain at least one of transition metal ions or rare earth ions as fluorescent ions. Above all, the near-infrared phosphor 31 preferably contains transition metal ions as fluorescent ions. Thus, the near-infrared phosphor 31, which is easy to obtain or synthesize, can be utilized. Therefore, the near-infrared light-emitting device 1 of high practicality can be provided. The transition metal ion may be at least one ion selected from the group consisting of $Ti^{3+}$, $V^{4+}$, $Cr^{4+}$, $V^{3+}$, $Cr^{3+}$, $V^{2+}$, $Mn^{4+}$, $Fe^{3+}$, $Co^{3+}$, $Co^{2+}$ and $Ni^{2+}$. Above all, the transition metal ion is preferably at least one ion selected from the group consisting of $Cr^{3+}$, $Cr^{4+}$ and $Ni^{2+}$.

The near-infrared phosphor 31 may be an oxide, sulfide, nitride, halide, oxysulfide, oxynitride, or oxy-halide. The near-infrared phosphor 31 may be at least one ion selected from the group consisting of halophosphate, phosphate, halosilicate, silicate, aluminate, aluminosilicate, borate, germanate, silicate nitride, aluminosilicate nitride, oxynitride silicate, and oxynitride aluminosilicate.

The $Cr^{3+}$-activated phosphor may be a composite oxide phosphor having a garnet-type crystal structure with many practical results. The $Cr^{3+}$-activated garnet phosphor may be at least one selected from the group consisting of $Y_3Al_2(AlO_4)_3:Cr^{3+}$, $La_3Al_2(AlO_4)_3:Cr^{3+}$, $Gd_3Al_2(AlO_4)_3:Cr^{3+}$, $Y_3Ga_2(AlO_4)_3:Cr^{3+}$, $La_3Ga_2(AlO_4)_3:Cr^{3+}$, $Gd_3Ga_2(AlO_4)_3:Cr^{3+}$, $Y_3Sc_2(AlO_4)_3:Cr^{3+}$, $La_3Sc_2(AlO_4)_3:Cr^{3+}$, $Gd_3Sc_2(AlO_4)_3:Cr^{3+}$, $Y_3Ga_2(GaO_4)_3:Cr^{3+}$, $La_3Ga_2(GaO_4)_3:Cr^{3+}$, $(Gd,La)_3Ga_2(GaO_4)_3:Cr^{3+}$, $Gd_3Ga_2(GaO_4)_3:Cr^{3+}$, $Y_3Sc_2(GaO_4)_3:Cr^{3+}$, $La_3Sc_2(GaO_4)_3:Cr^{3+}$, $Gd_3Sc_2(GaO_4)_3:Cr^{3+}$, and $(Gd,La)_3(Ga,Sc)_2(GaO_4)_3:Cr^{3+}$. The $Cr^{3+}$-activated garnet phosphor may be a solid solution having the phosphors as end components.

The near-infrared phosphor 31 may include a near-infrared phosphor in which at least chromium ions functioning as fluorescent ions are added to a crystal of an inorganic compound having the same spinel-type crystal structure as the compound $LiGa_5O_8$. The near-infrared phosphor emits fluorescence when irradiated with excitation light having a wavelength of 450 nm, and the spectral distribution of the fluorescence may have at least one fluorescence peak having a half-width of 120 nm or more within a wavelength range of 700 nm or more, and 1600 nm or less. Such a near-infrared phosphor emits fluorescence having a broad spectrum in any of the near-infrared wavelength regions of 700 nm or more, and 1600 nm or less, and can be used independently as a near-infrared phosphor in the near-infrared light-emitting device 1. Therefore, the near-infrared phosphor is suitable for the near-infrared light-emitting device 1 used for near-infrared spectroscopy. The half-width of the spectrum may be 140 nm or more, 160 nm or more, 180 nm or more, or 200 nm or more. The half-width of the spectrum may be 240 nm or less, or 220 nm or less. At least one fluorescence peak having the half-width may be one, two, or three or more fluorescence peaks.

Whether or not the near-infrared phosphor 31 has the same spinel-type crystal structure as the compound $LiGa_5O_8$ can be determined by measuring an X-ray diffraction pattern using an X-ray diffraction method.

Specifically, this means that the XRD pattern of the near-infrared phosphor is substantially the same as the XRD pattern of the compound $LiGa_5O_8$ (registration number: 33716) registered in ICSD. In addition, the near-infrared phosphor may contain nickel ions (described below) functioning as fluorescent ions in the crystal of the inorganic compound. In addition, the near-infrared phosphor may contain only chromium as fluorescent ions in the crystal of the inorganic compound.

The near-infrared phosphor may contain at least nickel ions functioning as fluorescent ions added to the crystal of the inorganic compound having the same spinel-type crystal structure as the compound $LiGa_5O_8$. With this configuration, the near-infrared phosphor can emit fluorescence having a maximum fluorescence intensity within a wavelength range of 1000 nm or more, and 1600 nm or less. Further, the near-infrared phosphor may have a small ratio of light-emitting component of the emission line, and a large fluorescence intensity. Further, the near-infrared phosphor may have a large internal quantum efficiency (IQE) in a wavelength region of 1000 to 1600 nm, and a large ratio of the IQE in a wavelength region of 1000 to 1600 nm to the total IQE. Therefore, the near-infrared phosphor may provide a near-infrared light-emitting device 1 that is advantageous for emitting light in a wavelength region of 1000 to 1600 nm. In the near-infrared phosphor, nickel ions functioning as fluorescent ions need not be added to the crystal of the inorganic compound.

The spinel-type crystal having the same spinel-type crystal structure as the compound $LiGa_5O_8$ may contain an alkali metal of a group 1 element, at least one of a group 13 element or scandium, and oxygen. That is, when the phosphor is expressed by the chemical formula $A_y(D_{1-x}Cr_x)_5O_8$, A may contain an alkali metal as a main component, and D may contain at least one of a group 13 element or scandium as a main component. D may contain a group 13 element as a main component. In such a composition, a crystal similar to $LiGa_5O_8$ in chemical properties is used. Therefore, it is expected that a near-infrared phosphor exhibiting fluorescence characteristics similar to those of $LiGa_5O_8$:$Cr^{3+}$, which has been found to exhibit a unique fluorescence spectrum shape and thermal quenching as a near-infrared phosphor, can be obtained.

It should be noted that A containing an alkali metal as a main component means that A contains an alkali metal of 75% by mole or more. A may contain an alkali metal of 90% by mole or more, 95% by mole or more, or 100% by mole. D containing at least one of the group 13 element or scandium as a main component means that D contains 75% by mole or more of at least one of the group 13 element or scandium. D may contain 90% by mole or more, 95% by mole or more, or 100% by mole of at least one of the group 13 element or scandium.

The alkali metal is preferably at least one element selected from the group consisting of Li, Na, K, Rb, and Cs, and is more preferably at least one element selected from the group consisting of Li, Na, and K. The group 13 element is preferably at least one element selected from the group consisting of B, Al, Ga, and In, and is more preferably Ga. The $LiAl_5O_8$ phosphor, which has the same spinel-type crystal structure as that of $LiGa_5O_8$, and to which at least one of Cr or Ni is added, emits fluorescence at a shorter wavelength than that of a $LiGa_5O_8$ phosphor. Therefore, the $LiAl_5O_8$ phosphor can be sensed by a relatively inexpensive Si-based sensor without using a relatively expensive InGaAs-based sensor.

It is preferable that the crystal mainly contains $LiGa_5O_8$, and a part of Ga is substituted with Cr. Here, "the crystal mainly contains $LiGa_5O_8$" means that the crystal contains 75% by mole or more of $LiGa_5O_8$, and it is preferable that the crystal contains 90% by mole or more of $LiGa_5O_8$. It is also preferable that the crystal mainly contains $LiGa_5O_8$, and a part of Ga is substituted by Cr and Ni.

The crystal above contains preferably a reduced alkali metal content relative to the stoichiometric composition. Specifically, it is preferable that the crystal has a composition deficient in element A relative to the stoichiometric composition $A(D_{1-x}Cr_x)_5O_8$. In this manner, a crystal similar in chemical properties to $LiGa_5O_8$ is used as the near-infrared phosphor. Therefore, unique fluorescence characteristics similar to those of an $LiGa_5O_8$:$Cr^{3+}$ near-infrared phosphor can be expected. When applied to spectroscopy, highly accurate non-destructive measurement can be expected.

The near-infrared phosphor is represented by the general formula $A_y(D_{1-x}Cr_x)_5O_8$. A mainly contains an alkali metal, D mainly contains at least one of a group 13 element or scandium, and y is preferably smaller than one. When y is smaller than one, a near-infrared phosphor with a small light emission component ratio in the emission line can be obtained. y is more preferably 0.90 or less, more preferably 0.85 or less, or particularly preferably 0.83 or less. Further, y is preferably 0.70 or more. When y is 0.70 or more, $\beta$-$Ga_2O_3$:$Cr^{3+}$ phosphor in different phase does not easily coexist, and desired unique fluorescence characteristics are easily obtained. y is more preferably 0.75 or more, or more preferably 0.77 or more. Here, x is preferably greater than 0.005, more preferably greater than 0.01, even more preferably greater than 0.02, or particularly preferably greater than 0.03. x is preferably less than 0.07, more preferably less than 0.06, even more preferably less than 0.05, or particularly preferably less than 0.04.

The near-infrared phosphor is preferably represented by the general formula $A_y(D_{1-(x+z)}Cr_xNi_z)_5O_8$. A and D as well as x and y are the same as those described above, and descriptions thereof is omitted. z is preferably greater than or equal to 0, more preferably greater than 0.005, more preferably greater than 0.01, particularly preferably greater than 0.02, or most preferably greater than 0.03. Further, z is preferably less than 0.07, more preferably less than 0.06, more preferably less than 0.05, or particularly preferably less than 0.04. (x+z) is preferably greater than 0.01, more preferably greater than 0.02, even more preferably greater than 0.025, or particularly preferably greater than 0.03. (x+z) is preferably less than 0.25, more preferably less than 0.10, or even more preferably less than 0.05. (x+z) may be less than 0.04. x may be greater than z, less than z, or the same as z. Although $A_y(D_{1-(x+z)}Cr_xNi_z)_5O_8$ is described in the composition formula, the valence of Ni is not limited, and Ni may have any of 0, +1, +2, +3, or +4.

The wavelength converter 30 may include only a near-infrared phosphor 31 as a phosphor. The near-infrared phosphor 31 may be a single near-infrared phosphor or a combination of a plurality of different near-infrared phosphors. When the wavelength converter 30 includes only a single near-infrared phosphor 31, not only is the configuration of the wavelength converter 30 simple, but also only the light component of the primary light 11, whose spectral half-width is generally narrow, can be converted into near-infrared rays. Therefore, the near-infrared light-emitting device 1 is excellent in industrial productivity, and can be configured to emit only near-infrared rays. However, when the wavelength converter 30 includes a plurality of different near-infrared phosphors 31, the spectral distribution of the near-infrared light component of the output light 50 can be easily controlled.

The wavelength converter 30 may further include a visible phosphor (not illustrated). The visible phosphor may be at least one phosphor selected from the group consisting of a green phosphor, a yellow phosphor, an orange phosphor, and a red phosphor. When the wavelength converter 30 includes the near-infrared phosphor 31 and a visible phosphor, the visibility of an object irradiated with the output light 50 can be ensured or enhanced. The green phosphor emits light having a maximum fluorescence intensity within a wavelength range of 490 nm or more, and less than 570 nm. The yellow phosphor emits light having a maximum fluorescence intensity within a wavelength range of 570 nm or more, and less than 585 nm. The orange phosphor emits light having a maximum fluorescence intensity within a wavelength range of 585 nm or more, and less than 620 nm. The red phosphor emits light having a maximum fluorescence intensity within a wavelength range of 620 nm or more, and less than 700 nm.

As illustrated in FIG. 1, the wavelength converter 30 may further include a sealing material 32 for sealing the near-infrared phosphor 31. The sealing material 32 may be at least one of an organic material or an inorganic material. Examples of the organic material include a transparent or translucent organic material such as silicone resin. Examples of the inorganic sealing material 32 include a transparent or translucent inorganic material such as low-melting-point glass. The wavelength converter 30 including the near-infrared phosphor 31 and the sealing material 32 is described, but the wavelength converter 30 need not include the sealing material 32.

The wavelength converter 30 may include a flat light output surface for outputting near-infrared rays. In this manner, the output light 50 including the near-infrared rays is output from the flat surface whose surface area is reduced. Therefore, the adhesion of dust or the like to the light output surface is prevented, and it is also convenient for cleaning or other operations. The flat light output surface means that the surface does not have irregularities caused by curvature.

The wavelength converter 30 may be formed in the shape of a plate or a sheet. In this manner, the wavelength converter 30 has a shape that extends horizontally with respect to a thickness direction. Therefore, the diffused light 21 of the primary light 11 emitted by the solid-state light-emitting element 10 can be incident on the wavelength converter 30 over a wide area. As a result, the near-infrared light-emitting device 1 can emit the output light 50 mainly composed of near-infrared rays with high output.

A gap may be provided between the light diffuser 20 and the wavelength converter 30. In the near-infrared light-emitting device 1 configured in this manner, the light diffuser 20 and the wavelength converter 30 are arranged apart. Therefore, the diffused light 21 of the primary light 11 diffused from the light diffuser 20 can be further diffused in the gap and incident on the wavelength converter 30. Therefore, the output light 50 can be further homogenized.

The light diffuser 20 and the wavelength converter 30 may be in direct contact with each other without a gap. In the near-infrared light-emitting device 1 configured in this manner, the light diffuser 20 and the wavelength converter 30 are arranged without being apart. Therefore, the light diffuser 20 and the wavelength converter 30 can be arranged close to each other, so that the near-infrared light-emitting device 1 can be formed in low profile.

As illustrated in FIG. 1, when the near-infrared light-emitting device 1 is viewed from the front of the light output surface from which the output light 50 is emitted, the light diffuser 20 may be arranged to cover the outline of a main light-extraction surface of the solid-state light-emitting element 10, and the wavelength converter 30 may be arranged to cover an entire light diffuser 20. In this manner, most of the primary light 11 emitted from the solid-state light-emitting element 10 is diffused from the light diffuser 20, and a large amount of the diffused light 21 is easily converted into near-infrared rays by the wavelength converter 30. Therefore, efficiency of converting the power input to the solid-state light-emitting element 10 or the solid-state light-emitting elements 10 which are arranged in an array form into near-infrared rays, can be enhanced. Here, the outline of the main light-extraction surface of the solid-state light-emitting element 10 means, for example, the outline of main light-extraction surfaces arranged in an array when the solid-state light-emitting element 10 is arranged in an array.

The near-infrared light-emitting device 1 according to the present embodiment can output the output light 50 including a near-infrared light component having a homogeneous distribution on the light output surface. The homogeneous near-infrared light component can also be emitted from the light output surface having a relatively large area. The near-infrared light-emitting device 1 is advantageous for a high-precision near-infrared spectroscopic analysis, since an object to be irradiated, such as a relatively large inspection object, can be illuminated by the homogeneous near-infrared rays. The near-infrared light-emitting device 1 according to the present embodiment can be used for electronic equipment, near-infrared spectroscopy, or the like, as will be described below.

The near-infrared light-emitting device 1 according to the present embodiment can be used for methods such as an inspection method, a detection method, a monitoring method, a sorting method, an analysis method, a measurement method, an evaluation method, or the like.

Electronic equipment

Next, electronic equipment according to the present embodiment will be described. The electronic equipment according to this embodiment includes the near-infrared light-emitting device 1 described above. In the electronic equipment configured in this manner, the diffused light 21 is absorbed by the near-infrared phosphor 31, and the diffused light 21 is converted into near-infrared rays. Therefore, the electronic equipment according to this embodiment can emit near-infrared rays having a homogeneous intensity distribution in the light output surface.

The electronic equipment can be used for any one of medicine, animal medicine, biotechnology, agricultural, forestry, and fisheries industries, livestock industry (for meat, meat products, dairy products, or the like), or industrial use (for contamination inspection, content inspection, shape inspection, packaging condition inspection, or the like). The electronic equipment can be used for inspection of any one of medical product, animal experiment, food, beverage, agricultural, forestry, and fisheries products, livestock product, or industrial product.

The electronic equipment can be used for any of humans, animals, plants, or objects. The electronic equipment can also be used for any of gases, liquids, and solid substance.

The electronic equipment may be, for example, medical equipment, therapeutic equipment, beauty equipment, health equipment, nursing care equipment, analytical equipment, measurement equipment, or evaluation equipment.

For example, for the purpose of medical treatment or biotechnology development, the electronic equipment according to this embodiment can be used for inspection, detection, measurement, evaluation, assay, analysis, observation, monitoring, separation, diagnosis, treatment, purification, or the like of 1) blood, body fluids, and components thereof, 2) excreta (urine and feces), 3) proteins and amino acids, 4) cells (including cancer cells), 5) genes, chromosomes, and nucleic acids, 6) biological samples, bacteria, specimens, and antibodies, 7) biological tissues, organs, and blood vessels, and 8) skin diseases and alopecia.

Further, for the purpose of beauty and health care, for example, the electronic equipment according to the present embodiment can be used for inspection, detection, measurement, evaluation, assay, analysis, observation, monitoring, beautification, sanitation, growth promotion, health promotion, diagnosis, or the like of 1) skin, 2) hair and body hair, 3) the oral cavity, dental cavity, periodontal cavity, 4) ears and nose, and 5) vital signs.

Further, for the purpose of use in, for example, agricultural, forestry, fisheries industries, livestock industry, or manufacturing industries, the electronic equipment of this embodiment can be used for inspection, detection, measurement, measurement, evaluation, assay, analysis, observation, monitoring, recognition, selecting, or sorting out of 1) industrial products (including electronic components and devices), 2) agricultural products (such as fruits and vegetables), 3) enzymes and bacteria, 4) marine products (such as fish, shellfish, crustaceans, and mollusks), 5) pharmaceuticals and biological samples, 6) foods and beverages, 7) the state of existence and conditions of people, animals, and things, 8) conditions of gases (including water vapor), 9) liquids, fluids, water, moisture, and humidity, 10) shapes, colors, internal structures, and physical conditions of things, 11) spaces, positions, and distances, 12) the contamination status of things, 13) conditions of molecules and particles, and 14) industrial wastes.

In addition, for example, for the purpose of nursing care, the electronic equipment according to the present embodiment can be used for, for example, excretion confirmation, identification, management, and monitoring of health conditions.

As described above, the electronic equipment according to the present embodiment can be used for all applications such as inspection, detection, gauging, measurement, evaluation, assay, analysis, observation, monitoring, beautification, sanitation, growth promotion, health promotion, separation, diagnosis, treatment, purification, recognition, selection, separation, identification, management, or the like.

Sensing System and Near-Infrared Spectroscopy

Next, a sensing system and a near-infrared light-emitting device 1 according to this embodiment will be described. The sensing system includes the near-infrared light-emitting device 1. The near-infrared spectroscopy utilizes the near-infrared light-emitting device 1. The near-infrared light-emitting device 1 can emit near-infrared light having a homogeneous intensity distribution in the light output surface. Therefore, the near-infrared spectroscopy can be highly accurate.

The sensing system may include a detector for detecting near-infrared rays. The detector may be configured to irradiate an object to be irradiated with the output light 50 emitted from the near-infrared light-emitting device 1, and to detect the reflected or transmitted near-infrared component. A quantum type photodetector such as a photodiode, a phototransistor, a photo IC, a CCD image sensor, a CMOS image sensor, or the like, can be used as the detector. That is, the sensing system may include a near-infrared sensor or a near-infrared camera. The sensing system may include, for example, the near-infrared light-emitting device 1, a near-infrared sensor or a near-infrared camera, and an output device for outputting data obtained by the near-infrared sensor or the camera. The output device may be a television monitor, a printer, or the like.

The sensing system may be a transmission type spectroscopic device as illustrated in FIG. 2. A spectroscopic device 100 in a transmission type includes the near-infrared light-emitting device 1 and a spectrometer 120 as illustrated in FIG. 2. The spectrometer 120 includes a detector. An inspection object 110 is arranged between the near-infrared light-emitting device 1 and the spectrometer 120. The near-infrared light-emitting device 1 is configured to emit the output light 50. The inspection object 110 is arranged to be irradiated with the output light 50. The spectrometer 120 is arranged to receive, among the output light 50 irradiated on the inspection object 110, transmission light 111 transmitted through the inside of the inspection object 110, especially transmitted near infrared light. The spectrometer 120 detects and spectrally disperses the transmission light 111. The spectrometer 120 may be, for example, a near-infrared spectrometer.

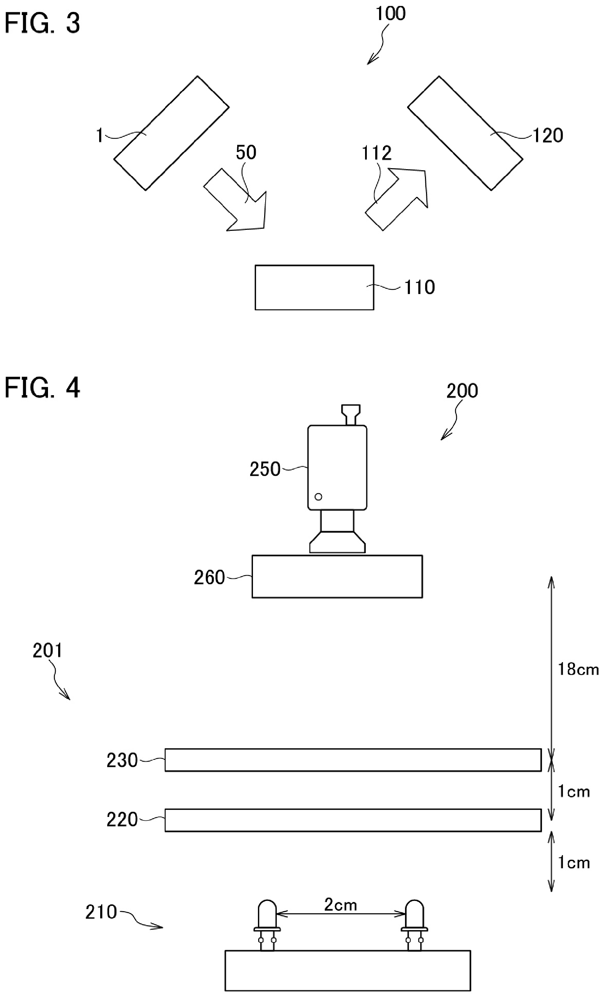

The sensing system may be a reflection type spectrometer, as illustrated in FIG. 3. The reflection type spectroscopic device 100 also includes the near-infrared light-emitting device 1 and the spectrometer 120, as illustrated in FIG. 3. However, in the reflection type spectroscopic device 100, the spectrometer 120 is arranged to receive reflection light 112 reflected by the inspection object 110, particularly near-infrared reflection light, out of the output light 50 irradiated on the inspection object 110. Other aspects are the same as those of the spectroscopic device 100 in a transmission type, and the description thereof will be omitted.

The sensing system and the near-infrared spectroscopy can be used for the same purpose as the electronic equipment described above. For example, the sensing system may be used as a food or industrial product quality control system, or the sensing system may be used as a plastic identification system.

Supplementary Notes

The description of the embodiments above discloses the following technology.

Technology 1

A near-infrared light-emitting device includes at least one solid-state light-emitting element emitting primary light having a maximum value of spectral intensity within a wavelength range of 380 nm or more, and less than 700 nm; a light diffuser that diffuses the primary light to emit diffused light; and a wavelength converter including a near-infrared phosphor that absorbs the diffused light and converts the diffused light into near-infrared rays having a maximum fluorescence intensity within a wavelength range of 700 nm or more, and less than 2500 nm. The near-infrared light-emitting device is configured to emit output light containing the near-infrared rays as a light component.

With this configuration, the primary light, which includes visible light with a strong directivity, is emitted from the solid-state light-emitting element, and can be diffused by using an orthodox light diffuser having a function of diffusing visible light. Further, the diffused light obtained by diffusing the primary light is absorbed by the near-infrared phosphor, and the diffused light is converted into near-infrared rays. Therefore, near infrared light having a homogeneous intensity distribution in a light output surface due to a light scattering effect can be emitted from a relatively large area. This allows the near-infrared light-emitting device according to this embodiment to emit near infrared light having a homogeneous intensity distribution in the light output surface.

Technology 2

The near-infrared light-emitting device according to Technology 1, in which the at least one solid-state light-emitting element is configured as a plurality of solid-state light-emitting elements, and the plurality of solid-state light-emitting elements are each arranged at an average interval of less than 100 cm. With this configuration, a plurality of solid-state light-emitting elements are arranged close to each other. Therefore, the intensity unevenness of the primary light in any surface can be further reduced. As a result, the diffused light emitted from the light diffuser can be homogenized to emit homogeneous near-infrared rays.

Technology 3

The near-infrared light-emitting device according to Technology 1 or 2, in which the light diffuser mainly contains a resin. The light diffuser configured in this manner is easy to mold and process, and there are many technical choices for materials and methods. Therefore, the near-infrared light-emitting device has a high industrial productivity, and can easily respond to customer demands.

Technology 4

The near-infrared light-emitting device according to any one of technologies 1 to 3, in which the diffused light includes light having a maximum spectral intensity within a wavelength range of 380 nm or more, and less than 700 nm, and does not include near-infrared rays converted by the near-infrared phosphor. With this configuration, the light diffuser does not emit near-infrared rays originating from a phosphor. Therefore, the spectral distribution of the output light can be simplified, and fluorescence interference can be mitigated between different types of phosphors. Further, the structure of the light diffuser can be simplified, so that the near-infrared light-emitting device with high industrial productivity can be provided without requiring advanced technology or a large number of man-hours.

Technique 5

The near-infrared light-emitting device according to any one of technologies 1 to 4, in which the light diffuser does not contain a phosphor. With this configuration, the light diffuser does not emit near-infrared rays originating from a phosphor. Therefore, the spectral distribution of the output light can be simplified, and fluorescence interference can be mitigated between different types of phosphors. Further, the structure of the light diffuser can be simplified, so that the near-infrared light-emitting device with high industrial productivity can be provided without requiring advanced technology or a large number of man-hours.

Technology 6

The near-infrared light-emitting device according to any one of technologies 1 to 5, in which the light diffuser includes a flat light emitting surface to emit the diffused light. In this manner, the surface of the wavelength converter facing the light diffuser becomes flat. Therefore, when the light diffuser and the wavelength converter are bonded to be integrated, it is possible to obtain a thin near-infrared light-emitting device.

Technology 7

The near-infrared light-emitting device according to any one of technologies 1 to 6, in which the wavelength converter includes a flat light output surface to output the near-infrared rays.

Technology 8

The near-infrared light-emitting device according to any one of technologies 1 to 7, in which the wavelength converter is formed in the shape of a plate or a sheet. In this manner, the wavelength converter has a shape that extends horizontally with respect to a thickness direction. Therefore, the diffused light of the primary light emitted from the solid-state light-emitting element can be incident on the wavelength converter over a wide area. As a result, the near-infrared light-emitting device can emit the output light mainly composed of near-infrared rays with high output.

Technology 9

The near-infrared light-emitting device according to any one of technologies 1 to 8, in which the wavelength converter includes only a near-infrared phosphor as a phosphor. With this configuration, not only the configuration of the wavelength converter is simple, but also only the light component of the primary light whose spectral half-width is generally narrow can be converted into near-infrared rays. Therefore, the near-infrared light-emitting device is excellent in industrial productivity, and can be configured to emit only near-infrared rays.

Technology 10

The near-infrared light-emitting device according to any one of technologies 1 to 9, in which the near-infrared phosphor includes transition metal ions as fluorescent ions. With this configuration, the near-infrared phosphor, which is easy to obtain or synthesize, can be utilized. Therefore, the near-infrared light-emitting device of high practicality can be provided.

Technology 11

Electronic equipment including the near-infrared light-emitting device according to any one of technologies 1 to 10. With this configuration, the diffused light is absorbed by the near-infrared phosphor, and the diffused light is converted into near-infrared rays. Therefore, the electronic equipment according to this embodiment can emit near-infrared rays having a homogeneous intensity distribution in the light output surface.

[Example]

Hereinafter, the present embodiments will be described in more detail with reference to Example and Comparative Examples, but the present embodiment is not limited to Example.

Example

As illustrated in FIG. 4, an evaluation device 200 was assembled, and output light emitted from a near-infrared light-emitting device 201 was photographed by a Vis-SWIR camera 250 (STC-LBS132U3V-SWIR, manufactured by OMRON Corporation: imaging bandwidth from 400 nm to 1700 nm). A visible cut filter 260 (sharp cut filter R70, manufactured by HOYA CORPORATION) was attached to the Vis-SWIR camera 250 to cut visible light emitted from the near-infrared light-emitting device 201. A filter used for the visible cut filter 260 has a transmission limit wavelength $\lambda T$ of $700 \pm 10$ nm, which is the midpoint between a wavelength with a transmittance of 5% and a wavelength with a transmittance of 72%, and cuts light with a wavelength of less than 700 nm.

The near-infrared light-emitting device 201 according to Example includes a solid-state light-emitting element 210, a light diffuser 220, and a wavelength converter 230. The solid-state light-emitting element 210, the light diffuser 220, and the wavelength converter 230 were arranged in this order. Two blue LEDs (LC303PBL1-30Q, dominant wavelength 470 nm, manufactured by Kingbright Electronic Co., Ltd.) were used for the solid-state light-emitting element 210. Then, the two blue LEDs were attached to a substrate so that the distance between the blue LEDs was 2 cm. A diffuser plate SW-12, manufactured by Nikon Corporation was used for the light diffuser 220. The wavelength converter 230, which was manufactured as described below, was used. The distance between a tip of the blue LED and a surface of the light diffuser 220 facing the solid-state light-emitting element 210 was set to 1 cm. The distance between the center of the light diffuser 220 and the center of the wavelength converter 230 was set to 1 cm. The distance between the center of the wavelength converter 230 and the center of the visible cut filter 260 was set to 18 cm.

The wavelength converter 230 was prepared as described below.

Preparation of Raw Materials

First, the following compound powders were prepared as raw materials.

- Lithium carbonate ($Li_2CO_3$): purity 2N, manufactured by FUJIFILM Wako Pure Chemical Corporation
- Gallium oxide ($Ga_2O_3$): purity 4N, manufactured by Nippon Rare Metal, Inc.
- Chromium oxide ($Cr_2O_3$): purity 3N, manufactured by Kojundo Chemical Laboratory Co., Ltd.
- Nickel oxide (NiO): purity 3N, manufactured by FUJIFILM Wako Pure Chemical Corporation

Preparation of Phosphor

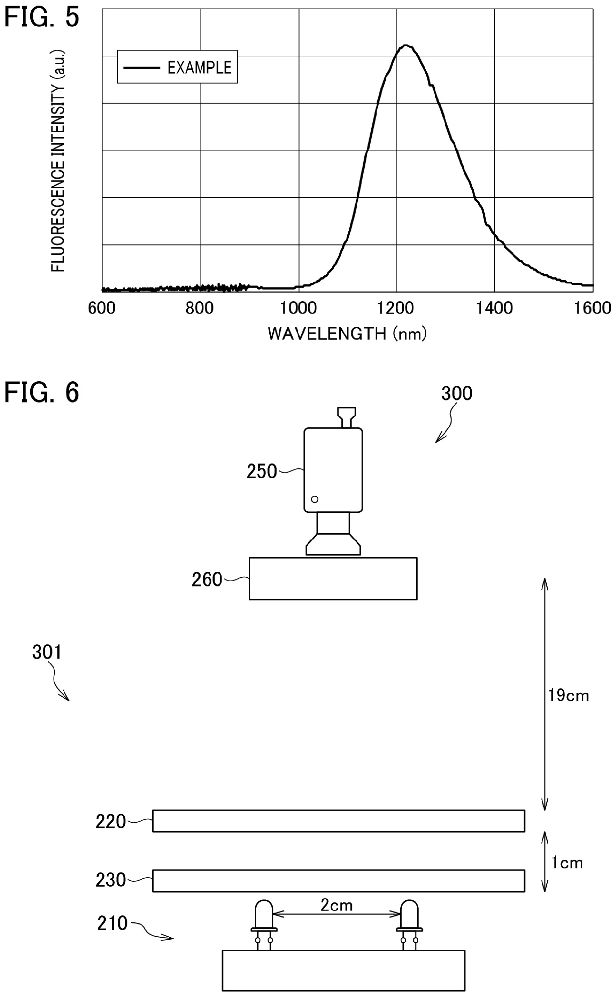

Compound powders were weighed to prepare a compound of $Li(Ga_{0.94}Cr_{0.05}Ni_{0.01})_5O_8$, and the mixed raw materials were dry-mixed using a mortar and pestle. The mixed raw materials were transferred to an alumina crucible with a lid, and fired at 1400°C for 4 hours in the atmosphere using a box-type electric furnace. The fired product obtained by firing was lightly crushed to prepare a phosphor of $Li(Ga_{0.94}Cr_{0.05}Ni_{0.01})_5O_8$. Fig. 5 illustrates fluorescence spectrum of the phosphor.

Preparation of Wavelength converter

Next, 0.40 g of the phosphor described above and silicone resin (1 g of KER-2600A, 1 g of KER-2600B) were mixed and further defoamed using an agitating and defoaming machine. The agitating and defoaming machine, manufactured by Thinky Corporation, product name: Awatori Neritaro (registered trademark), model: ARE-310, was used. The rotation speed of the agitating and defoaming machine was set to about 2000 rpm, and a treatment was carried out for 3 minutes. In this manner, a phosphor paste containing the phosphor and silicone resin described above was prepared. The obtained phosphor paste was dropped into a frame having a height of about 320 $\mu$m using a dispenser (model: ML-5000XII, manufactured by Musashi Engineering, Inc.). The phosphor paste was then heated in the atmosphere at 150°C for 2 hours to cure. In this manner, a wavelength converter 230 having a length of 10 mm $\times$ width of 10 mm $\times$ thickness of about 300 $\mu$m was prepared.

Comparative Example 1

As illustrated in FIG. 6, an evaluation device 300 was assembled, and output light emitted from a near-infrared light-emitting device 301 was photographed using the Vis-SWIR camera 250.

The near-infrared light-emitting device 301 according to Example includes the solid-state light-emitting element 210, the light diffuser 220, and the wavelength converter 230. The solid-state light-emitting element 210, the wavelength converter 230, and the light diffuser 220 were arranged in this order. The distance between the tip of the blue LED and the surface of the wavelength converter 230 facing the solid-state light-emitting element 210 was set to about 0 cm. The distance between the center of the wavelength converter 230 and the center of the light diffuser 220 was set to 1 cm. The distance between the surface of the light diffuser 220 facing the visible cut filter 260 and the center of the visible cut filter 260 was set to 19 cm. In other aspects, the evaluation device 300 was assembled in the same manner as in Example.

Comparative Example 2

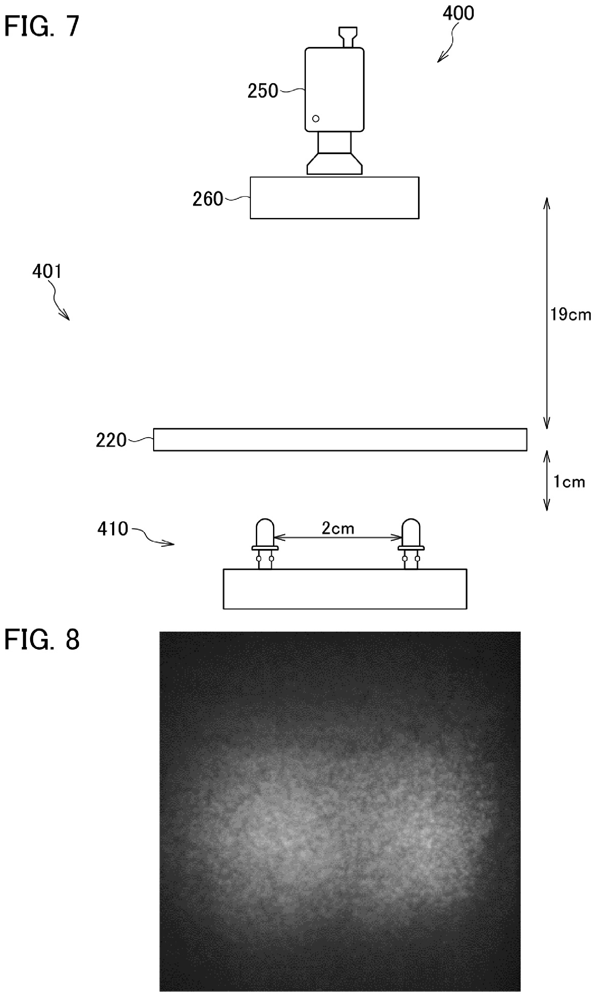

As illustrated in FIG. 7, an evaluation device 400 was assembled, and output light emitted from a near-infrared light-emitting device 401 was photographed using the Vis-SWIR camera 250.

The near-infrared light-emitting device 401 according to Example includes a solid-state light-emitting element 410 and the light diffuser 220. The solid-state light-emitting element 410 and the light diffuser 220 were arranged in this order. Two near-infrared LEDs (L13895-0145P, manufactured by Hamamatsu Photonics K.K., peak emission wavelength: 1450 nm) were used for the solid-state light-emitting element 410. The distance between the tips of the near-infrared LED and the surface of the light diffuser 220 facing the solid-state light-emitting element was set to 1 cm. The distance between the surface of the light diffuser 220 facing the visible cut filter and the center of the visible cut filter was set to 19 cm. In other aspects, the evaluation device 400 was assembled in the same manner as in Example.

As illustrated in FIG. 8, in the near-infrared light-emitting device 201 according to Example, primary light including visible light emitted from the solid-state light-emitting element 210 is diffused by the light diffuser 220, and then converted into near-infrared rays by the wavelength converter 230. Therefore, the output light of Example had a homogeneous intensity distribution in the light output surface.

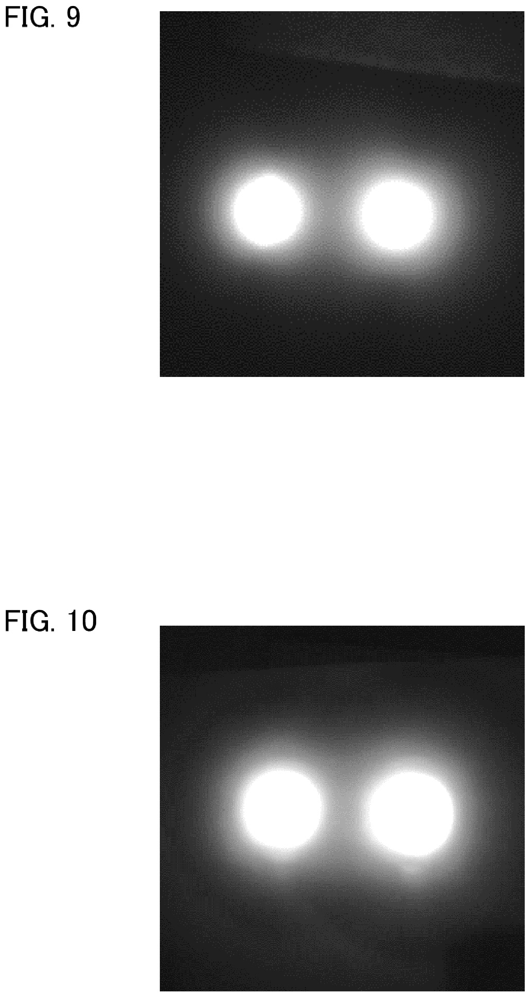

However, as shown in FIG. 9, in the near-infrared light-emitting device 301 according to Comparative Example 1, primary light including visible light, emitted from the solid-state light-emitting element 210, is converted into near-infrared rays by the wavelength converter 230, and then transmitted through the light diffuser 220. The general light diffuser 220 easily diffuses visible light. However, the light diffuser 220 does not easily diffuse near-infrared light having a long wavelength, and transmit near-infrared light with high directivity. Therefore, the output light including near-infrared rays emitted from the near-infrared light-emitting device 301 tends to have high intensity directly above LED chips, and low intensity between the LED chips. Therefore, the intensity distribution of the output light in Comparative Example 1 was not homogeneous in the light output surface as compared with the output light of Example.

Further, as shown in FIG. 10, in the near-infrared light-emitting device 401 according to Comparative Example 2, primary light including visible light emitted from the solid-state light-emitting element 410 passes through the light diffuser 220. Therefore, as in Comparative Example 1, the intensity distribution of the output light of Comparative Example 2 in the light output surface was not homogeneous as compared with the output light of Example.

Next, luminance saturation of the near-infrared phosphor used in Example was investigated as described below.

Production of Near-infrared light-emitting device

First, 0.8 g of powder of the mixed raw material described above was filled in a mold, and the mixed raw material powder was compressed with pressure of about 3 MPa using a hand press to produce a cylindrical molded body having a diameter of Φ13 mm.

Sintering of Mixed Raw Materials

A produced molded body was charged into an alumina shell (with a lid, Material SSA-S, manufactured by Nikkato Corporation), and the temperature was raised to 1400°C at a rate of 300°C/hour. After sintering in atmosphere for 4 hours, the molded body was cooled to room temperature at 300°C/hour to produce a ceramic sample.

Polishing of Sintered Sample

A produced ceramic sample was processed to a film thickness of 140 μm using an automatic grinder (DAG810, Disco Corporation).

Production of Near-infrared light-emitting device

The ceramic sample with a film thickness of 140 μm was cut to an LED chip size of 3.2 mm×2.6 mm using an automatic dicing saw (DAD3350, Disco Corporation). The cut ceramic sample was mounted on a blue LED chip to produce a near-infrared light-emitting device.

Measurement of Near-infrared Light Emission Intensity of Near-infrared light-emitting device

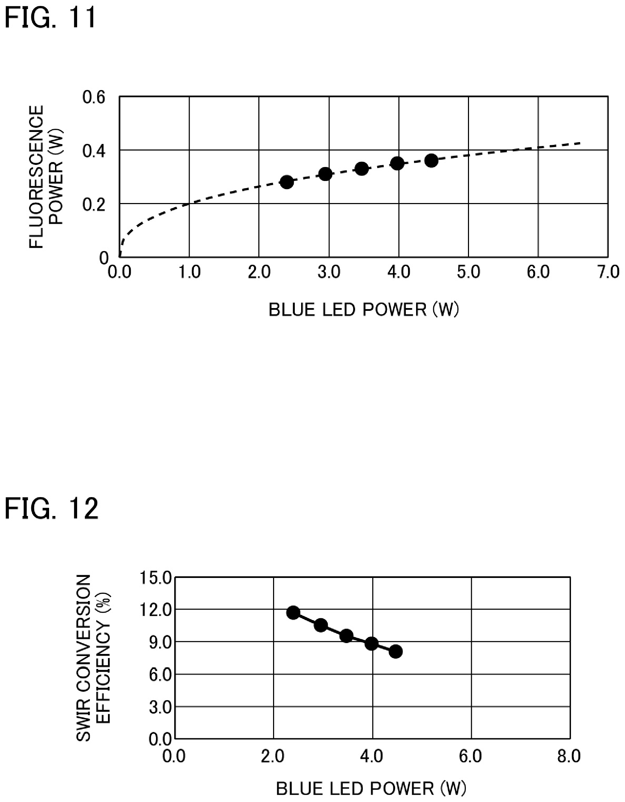

In order to cut blue light as excitation light and measure the output of near-infrared fluorescence, a visible light cut sheet was installed on a produced near-infrared light-emitting device. Then, electric current was applied to the blue LED starting from 0.4 A with a step width of 0.1 A, and the light output was measured with a power meter (PM100D, manufactured by Thorlabs, Inc.). The result is illustrated in FIG. 11.

Measurement of Luminance Saturation Constant of Near-infrared phosphor)

Fig. 11 illustrates the relationship between blue LED output and fluorescence output. α and y, which agree as much as possible with an index of $I = \alpha I_{uv}^{\gamma}$ (I is luminance of light emission, $I_{uv}$ is an excitation intensity, α is a constant, and y is a luminance saturation constant), were calculated. As a result, the constant α = 0.2 and the luminance saturation constant γ = 0.4 were derived.

Conversion Efficiency

Next, conversion efficiency was calculated by dividing the output of near-infrared fluorescence by the output of the blue LEDs, and represented using a graph. The result is illustrated in FIG. 12.

FIGS. 11 and 12 illustrate that the near-infrared phosphor used in Example has a luminance saturation constant of 0.4, and is a phosphor whose luminance is relatively easy to saturate. However, the wavelength converter 230 according to Example uses light of a relatively low output via the light diffuser 220 as excitation light. Therefore, even when a phosphor whose luminance is easy to saturate as in Example is used, the influence of luminance saturation can be relatively reduced.

The entire contents of Japanese Patent Application No. 2023-156367 (filed on September 21, 2023) are incorporated herein by reference.

The present embodiments have been described above, but the present embodiments are not limited thereto, and modifications can be made within the scope of the present embodiments.

INDUSTRIAL APPLICABILITY

According to the present disclosure, it is possible to provide a near-infrared light-emitting device which emits near-infrared rays having a homogeneous intensity distribution in a light output surface, and electronic equipment using the near-infrared light-emitting device.

REFERENCE SIGNS LIST

1    Near-infrared light-emitting device
10   Solid-state light-emitting element
11   Primary light
20   Light diffuser
21   Diffused light
30   Wavelength converter
31   Near-infrared phosphor
50   Output light

**Claims**

1. A near-infrared light-emitting device comprising:

   at least one solid-state light-emitting element emitting primary light having a maximum value of spectral intensity within a wavelength range of 380 nm or more, and less than 700 nm; a light diffuser that diffuses the primary light to emit diffused light; and a wavelength converter including a near-infrared phosphor that absorbs the diffused light and converts the diffused light into near-infrared rays having a maximum fluorescence intensity within a wavelength range of 700 nm or more, and less than 2500 nm, wherein the near-infrared light-emitting device is configured to emit output light containing the near-infrared rays as a light component.

2. The near-infrared light-emitting device according to claim 1, wherein

   the at least one solid-state light-emitting element is configured as a plurality of solid-state light-emitting elements, and the plurality of solid-state light-emitting elements are each arranged at an average interval of less than 100 cm.

3. The near-infrared light-emitting device according to claim 1 or 2, wherein the light diffuser mainly contains a resin.

4. The near-infrared light-emitting device according to any one of claims 1 to 3, wherein the diffused light includes light having a maximum spectral intensity within a wavelength range of 380 nm or more, and less than 700 nm, and does not include near-infrared rays converted by the near-infrared phosphor.

5. The near-infrared light-emitting device according to any one of claims 1 to 4, wherein the light diffuser does not contain a phosphor.

6. The near-infrared light-emitting device according to any one of claims 1 to 5, wherein the light diffuser includes a flat light emitting surface to emit the diffused light.

7. The near-infrared light-emitting device according to any one of claims 1 to 6, wherein the wavelength converter includes a flat light output surface to output the near-infrared rays.

8. The near-infrared light-emitting device according to any one of claims 1 to 7, wherein the wavelength converter is formed in the shape of a plate or a sheet.

9. The near-infrared light-emitting device according to any one of claims 1 to 8, wherein the wavelength converter includes only a near-infrared phosphor as a phosphor.

10. The near-infrared light-emitting device according to any one of claims 1 to 9, wherein the near-infrared phosphor includes transition metal ions as fluorescent ions.

11. Electronic equipment comprising the near-infrared light-emitting device according to any one of claims 1 to 10.

FIG. 1

FIG. 2

FIG. 3

FIG. 4

FIG. 5

FIG. 6

FIG. 7

400

250

260

401

19cm

220

1cm

410

2cm

FIG. 8

FIG. 9

FIG. 10

FIG. 11

FIG. 12

## INTERNATIONAL SEARCH REPORT

| International application No. |
|---|
| **PCT/JP2024/030777** |

### A. CLASSIFICATION OF SUBJECT MATTER

*H01L 33/58*(2010.01)i; *A61N 5/06*(2006.01)i; *C09K 11/62*(2006.01)i; *G02B 5/02*(2006.01)i; *G02B 5/20*(2006.01)i; *H01L 33/00*(2010.01)i; *H01L 33/50*(2010.01)i
FI:  H01L33/58; A61N5/06; C09K11/62; G02B5/02 B; G02B5/20; H01L33/00 H; H01L33/50

According to International Patent Classification (IPC) or to both national classification and IPC

### B. FIELDS SEARCHED

Minimum documentation searched (classification system followed by classification symbols)

H01L33/00-33/64; H01S5/00-5/50; A61N5/00-5/10; A61M36/10-36/14; C09K11/00-11/89; G02B5/00-5/136; G02B5/20-5/28; F21K9/00-9/90; F21S2/00-45/70; F21V8/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | WO 2023/022791 A1 (LUMILEDS LLC) 23 February 2023 (2023-02-23) paragraphs [0034]-[0068], [0082]-[0108], fig. 1-12 | 1-11 |
| Y | WO 2018/212070 A1 (FUJIFILM CORPORATION) 22 November 2018 (2018-11-22) paragraphs [0003], [0013]-[0044], [0097]-[0116], fig. 1, 9 | 1-11 |
| Y | JP 2019-61954 A (LG INNOTEK CO., LTD.) 18 April 2019 (2019-04-18) paragraphs [0049]-[0069], [0105]-[0125], fig. 1-2, 4-8 | 1-11 |
| A | US 2022/0229183 A1 (OPTONOMOUS TECHNOLOGIES, INC.) 21 July 2022 (2022-07-21) entire text, all drawings | 1-11 |

☐ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 October 2024** | **08 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

### INTERNATIONAL SEARCH REPORT
#### Information on patent family members

| International application No. |
| --- |
| **PCT/JP2024/030777** |

| Patent document cited in search report | | | Publication date (day/month/year) | Patent family member(s) | | | Publication date (day/month/year) |
| --- | --- | --- | --- | --- | --- | --- | --- |
| WO | 2023/022791 | A1 | 23 February 2023 | US | 2023/0064945 | A1 | |
| | | | | KR | 10-2024-0049587 | A | |
| | | | | CN | 118202019 | A | |
| WO | 2018/212070 | A1 | 22 November 2018 | (Family: none) | | | |
| JP | 2019-61954 | A | 18 April 2019 | US | 2019/0097094 | A1 | |
| | | | | paragraphs [0089]-[0108], [0144]-[0164], fig. 1-2, 4-8 | | | |
| | | | | US | 2021/0175395 | A1 | |
| | | | | EP | 3460844 | A2 | |
| | | | | EP | 3787024 | A1 | |
| | | | | EP | 4343865 | A2 | |
| | | | | KR | 10-2019-0035491 | A | |
| | | | | CN | 109616468 | A | |
| | | | | KR | 10-2022-0159325 | A | |
| | | | | CN | 117133763 | A | |
| | | | | KR | 10-2024-0024166 | A | |
| US | 2022/0229183 | A1 | 21 July 2022 | WO | 2020/243038 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015076872 A

- JP 2023156367 A